# EUROPEAN PATENT SPECIFICATION

(11) **EP 4 218 540 B1**
(45) Date of publication and mention of the grant of the patent: **04.11.2026**
(21) Application number: 22305081.6
(22) Date of filing: 26.01.2022
(51) Int. Cl.: A61B 3/00, G02C 7/02

(54) **METHOD FOR SIMULATING AN OPHTHALMIC LENS ON AN EYE OF A SUBJECT VIEWING A VIRTUAL THREE-DIMENSIONS SCENE USING A LIGHT FIELD DISPLAY**
VERFAHREN ZUR SIMULATION EINER OPHTHALMISCHEN LINSE AUF EINEM AUGE EINES SUBJEKTS ZUR BETRACHTUNG EINER VIRTUELLEN DREIDIMENSIONALEN SZENE UNTER VERWENDUNG EINER LICHTFELDANZEIGE
PROCÉDÉ DE SIMULATION D'UNE LENTILLE OPHTALMIQUE SUR L' OEIL D'UN SUJET VISUALISANT UNE SCÈNE VIRTUELLE EN TROIS DIMENSIONS À L'AIDE D'UN ÉCRAN À CHAMP DE LUMIÈRE

(43) Date of publication of application: 02.08.2023
(73) Proprietor: Essilor International, 94220 Charenton-le-Pont (FR)
(72) Inventor: BOUTINON, Stéphane, 75013 Paris (FR); HERNANDEZ-CASTANEDA, Martha, 75012 Paris (FR); BARANTON, Konogan, 77450 Montry (FR); PELOUX, Marius, 91120 Palaiseau (FR); MARIN, Gildas, 75012 Paris (FR)
(74) Representative: Jacobacci & Partners France

(56) References cited:
- WO-A1-2017/134275
- WO-A1-2021/022193
- CN-C- 1 220 040
- JP-A- 2000 107 129
- US-B2- 10 416 456
- US-B2- 7 914 148

## Description

### TECHNICAL FIELD OF THE INVENTION

This disclosure relates to a method for simulating ophthalmic correction of an eye of a subject viewing a virtual three-dimensions scene using a light field display.

More precisely the disclosure relates to a method for simulating an ophthalmic correction lens on an eye or two eyes of a subject viewing a three-dimensional scene.

### BACKGROUND INFORMATION AND PRIOR ART

Numerous documents describing methods for simulating an ophthalmic correction lens exist, such as simulators using virtual reality (VR) or augmented reality (AR). Most of the time, these solutions use rendering on each eye to reconstruct the three-dimensions thanks to convergence properties, breaking thus the accommodation convergence couple.

In addition, different methods to simulate the accommodation are known. For example, it is also known to use active optics coupled with virtual reality or augmented reality technologies to modulate a focalization plane. These methods calculate two or three accommodation states allowing correcting partly the eye refraction. However, these methods do not render properly the multiple possible positions of the focal plane in a real three-dimensions environment and thus do not allow to simulate correctly complex types of lenses, such as progressive addition lenses (PAL) or, hereinafter, progressive lenses.

For example, the simulation of a progressive addition lens using a virtual reality device is often consistent for simulating the distortion but not consistent for simulating lens power distribution. Specifically, the methods to simulate power lens distribution often use a plurality of two-dimensions blurred images, that break a coupling between accommodation and convergence. Consequently, these methods do not allow to obtain a simulation of what a retina would perceive with a real lens.

Thus, there is a need for a method enabling a simulation and evaluation of complex ophthalmic correction lenses and, in particular, a method able to provide proper binocular rendering with correct accommodation and convergence properties.

It is known from document US7914148B2 a method for simulating the optical effect of an ophthalmic lens design using a display projector, such as a liquid crystal display (LCD), to present an output image to a wearer, said output image being a 2D output image.

### SUMMARY OF THE INVENTION

Therefore one object of the disclosure is to provide a method for simulating an ophthalmic lens on an eye of a subject viewing a virtual three-dimensions scene using a light field display, said light field display comprising a light field window, said method comprising the following steps:
- receiving a set of input parameters comprising scene parameters of the virtual three-dimensions scene,
- calculating, by ray tracing, a virtual ray between a point of the virtual three-dimensions scene and a point of an eye pupil plane, the virtual ray passing through a virtual lens and being defined on the basis of a model providing a deviation angle of the virtual ray through the virtual lens, the deviation angle depending on at least one incidence angle and position of the virtual ray on the virtual lens, repeating the calculating by ray tracing for a plurality of virtual rays passing through the virtual lens and joining couples of one point of the virtual three-dimensions scene and another point of the eye pupil plane, so as to scan spatially and angularly the light field window;
- determining a light field representing a modified virtual three-dimensions scene based on the scene parameters and on the deviation angle associated to each virtual ray of the plurality of virtual rays;
- generating the light field representing the modified virtual three-dimensions scene on the light field window towards the eye pupil plane of the subject.

In the method according to the disclosure, the properties of the light field display allow defining positioning and direction of each light ray on its surface. These properties are used in the steps of the disclosure to allow a subject to view correctly a virtual three-dimensions scene without wearing an eyewear. Specifically, the disclosure proposes to simulate the dioptric corrections of an ophthalmic corrective lens by using a light field display.

Thus, according to the method of this disclosure, it is possible to calculate virtual rays between a light field display and a scene. These virtual rays are calculated on the whole surface of the light field display. By taking into account all the dioptric effects of an ophthalmic corrective lens, the method enables to simulate the proximity of an object through the virtual lens. Furthermore, by taking into account all the prismatic effects of the ophthalmic corrective lens, the method enables to simulate distortion effects when looking through the virtual lens. The simulation is thus more accurate. Such features allow maintaining a link between the convergence and the accommodation, which can be now considered in a same step, which is not the case in the prior art. For example, it is possible to associate to each ray of the light field display a proximity of an object in the virtual three-dimensions scene, which is very useful in the simulation of complex lens. Thus, the method according to this disclosure allows improving the simulation of the ophthalmic corrective lenses by providing a simulation that is closer to real life experience for a user.

The light field window may correspond to a field of view of at least 40 degrees in a horizontal plane and 40 degrees in a vertical plane and/or an angular resolution of less than 5 degrees.

In an embodiment, the light field window may extend spatially over a surface area of at least 3 mm in a horizontal plane and 3 mm in a vertical plane and/or present a spatial resolution of at least 0,5 dot/mm.

According to a particular aspect, the method according to this disclosure comprises a step of acquiring a three-dimensions image of the scene using a three-dimensions capturing device to extract the scene parameters, said scene parameters comprising geometric and/or photometric properties of the scene.

According to another particular aspect, the step of determining a light field comprises a step of applying the geometric and/or photometric properties of the scene.

Advantageously, the step of calculating and/or the step of generating according to the method of this disclosure comprise(s) a step of selecting a subset of the plurality of virtual rays passing through the pupil of the eye of the subject.

Advantageously, the model is a simulation model determined on the basis of geometrical optics calculations and/or wave optics calculations, and/or photometric optics calculation.

In an embodiment of this disclosure, the model comprises a shape of the lens and a positioning of the lens in relation to the eye.

Advantageously, the model comprises a distortion model of the virtual lens and/or a spherical power model of the virtual lens comprising at least a sphere parameter, and/or an astigmatism power model of the virtual lens comprising at least a cylinder and/or an axis parameters or J0 and J45 parameters and/or a filtering lens.

According to a particular aspect, the step of determining of the method according to this disclosure comprises a step of representing the modified virtual three-dimensions scene further comprising applying a convolution or image deformation, adapted for simulating blur, distortion, diffraction, chromatism, scattering or transmission attenuation.

According to a particular and advantageous embodiment, the method according to this disclosure is adapted for simulating an other ophthalmic lens on an other eye of the subject, said method comprising the steps of calculating, determining and generating for simulating the other ophthalmic lens for the other eye of the subject, said subject parameters in the step of receiving comprising other parameters among a pupil diameter of the other eye and/or a pupil position of the other eye relative to the light field display.

Advantageously, the virtual lens comprises a progressive power lens or a multifocal lens, a single vision lens, an eyeglass lens, a contact lens or a filter.

In an example, the virtual ray and the plurality of virtual rays in the step of calculating are configured to propagate from the eye pupil plane to the at least one object of the scene.

Alternatively, the virtual ray and the plurality of virtual rays in the step of calculating are configured to propagate from the at least one object of the scene to the eye pupil plane.

A further object of the disclosure is to provide a system comprising a calculator and a light field display, the system being adapted and configured to operate the method according to the disclosure.

Advantageously, the light field display according to the present disclosure presents a spatial resolution, which is defined as a number of pixels per unit distance in one direction of alignment.

According to a particular example, the light field window has a size, which corresponds to a surface of emission of the light field window.

The field of view per angular resolution means an angular step of emission. The angular resolution can be defined as the smallest angular step between two directions of emission of a given (macro)pixel of the light field display.

The ratio between the field of view and the angular resolution is equal to the number of directions of emission along the direction in which the field of view is defined (e.g. horizontal or vertical).

Preferably, the method according to the disclosure is configured to be implemented by a computer.

In the present document, a computer may be a processor, a calculation module or a calculator or a calculation unit. The steps of receiving, calculating, determining are implemented by a single calculation module or they are each implemented by separate calculation modules.

A further object of this disclosure is to provide a device configured to execute all the steps of the method according to this disclosure.

A further object of this disclosure is to provide a computer-program product comprising one or more stored sequences of instructions that are accessible to a processor, and which, when executed by the processor, causes the processor to carry out the method according to the disclosure.

A further object of the disclosure is to provide a computer readable medium carrying one or more sequences of instructions of the computer program product of the present disclosure.

### BRIEF DESCRIPTION OF THE DRAWINGS

The following description with reference to the accompanying drawings will make it clear what the disclosure consists of and how it can be achieved. The disclosure is not limited to the embodiments illustrated in the drawings. Accordingly, it should be understood that where features mentioned in the claims are followed by reference signs, such signs are included solely for the purpose of enhancing the intelligibility of the claims and are in no way limiting on the scope of the claims.

Reference is now made to the brief description below, taken in connection with the accompanying drawings and detailed description, wherein like reference numerals represent like parts.

In the accompanying drawings:
- Figure 1 shows a side view of a first type of light field display device according to the prior art;
- Figure 2 shows a side view of a second type of light field display device according to the prior art;
- Figures 3, 4, 5 show examples of a preliminary step of calculation used to build a distortion model;
- Figures 6, 7, 8 show examples of a preliminary step of calculation used to apply a spherical power model and astigmatism power model to a Light Field Display;
- Figure 9 shows a first embodiment of a method;
- Figure 10 shows a second embodiment of a method;
- Figure 11 shows a schematic side of view of a light field display used in the method;
- Figures 12, 13, 14 represent schematically an example of embodiment of a step of calculating and a step of determining of the method;
- Figure 15 represents schematically an exemplary embodiment of a step of generating of the method;
- Figure 16 shows a schematic representation of a step of selecting applied in the step of calculating and in the step of determining of the method;
- Figure 17 shows a schematic representation of a step of selecting applied in the step of generating of the method;
- Figure 18 shows a system according to the present disclosure allowing a subject to view a scene;
- Figure 19 represents schematically another exemplary embodiment of a step of generating of the method;
- Figure 20 represents schematically another exemplary embodiment of a step of generating of the method.

### DETAILED DESCRIPTION OF THE INVENTION

In the description which follows the drawings are not necessary to scale and certain features may be shown in generalized or schematic form in the interest of clarity and conciseness or for informational purposes. In addition, although making and using various embodiments are discussed in detail below, it should be appreciated that as described herein are provided many inventive concepts that may be embodied in a wide variety of contexts. Embodiments discussed herein are merely representative and do not limit the scope of the invention. It will also be obvious to one skilled in the art that all the technical features that are defined relative to a process can be transposed, individually or in combination, to a device and conversely, all the technical features that are defined relative to a device can be transposed, individually or in combination, to a process.

### Definitions

In the present document, a light field (LF) is a vector function that describes the light rays flowing in every direction through every point in space. This vector function may also embed information representing the spectrum of the light.

A conventional display device generates 2D images, wherein the light properties of the image depend on the position (x, y) of each point in an image. In contrast, a light field display device (or LFD) is arranged to generate a light field and enables to control the amount of light and color or spectral characteristics, not only as a function of the position (X, Y), but also as a function of at least one direction. Some light field display devices generate a 3D light field, wherein the amount of light and color or spectral characteristics depends on the position (x, y) in the image and on one direction (u). Generating a 3D light field (x, y, u) is also called 1D integral imaging.

Some light field displays generate a 4D light field in which the amount of light depends on the position (x, y) in the image and on two transverse directions, noted for example (u, v). In other words (x,y) correspond to some coordinates in one plane, (u,v) to some coordinates in another plane, and that one direction of a light ray is defined by a couple (x,y) and a couple (u,v). In general, the direction of the vector u is in a horizontal plane and the direction of the vector v is in a vertical plane. The vectors u and v can also be angular coordinates. Generating a 4D light field (x, y, u, v) is also called integral imaging. Light field display devices can generally control the position and the direction of the light rays for each color (for example RGB or red-blue-green in display devices based on the three colors).

For example, the light field display devices according to the current disclosure are similar to the light field display disclosed in the document WO2021/122640. All the properties of the light field display disclosed in this document are used in the present disclosure.

Ideally, a light field display device is like a window and enables to transmit light rays from each point of the image in independent directions.

Different kinds of light field display devices are available and may be used in the method and in the system according to the current disclosure.

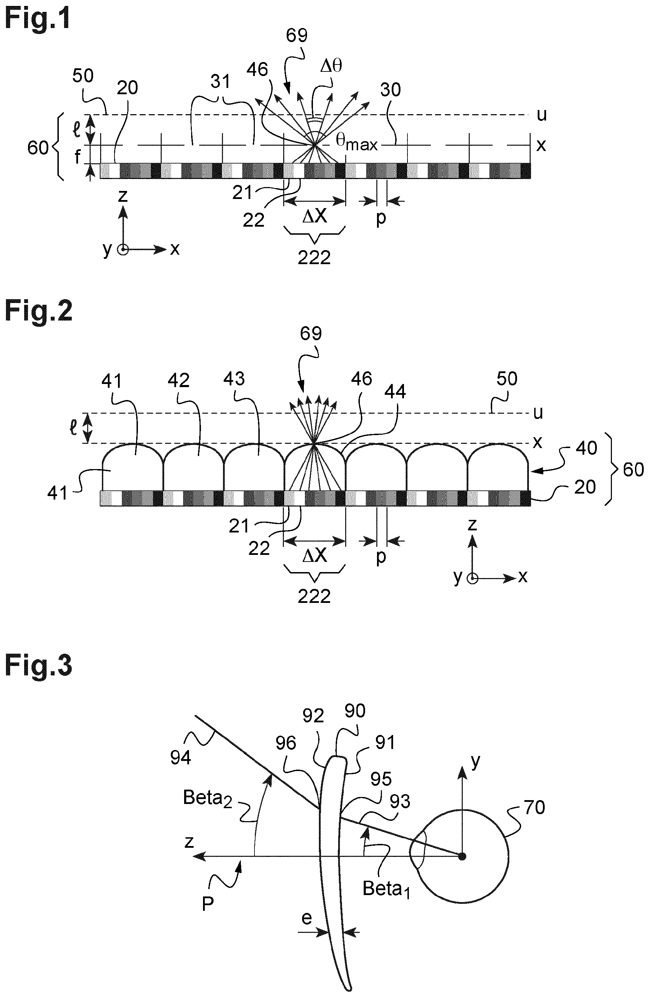

For example, figure 1 shows a side view of a known light field display 60. In this case, the light field display comprises a display panel 20 and a parallax barrier 30. In addition, the display panel simulated comprises pixels 21, 22... which can be addressed individually. The display panel 20 may include a line of pixels, or, more generally, a 2D array of pixels. In the example illustrated on figure 1, the pixels are positioned with a constant pitch, noted p, along axis X, in an orthogonal reference system (X, Y, Z). The pixels are grouped according to macropixels 222. A macropixel 222 is formed by adjacent pixels that are arranged to generate rays from a same point of the light field display at various angles. The rays of each macropixel 222 intersect in a same point 46 into the light field display 60 before propagating according to a pluralities of directions. As illustrated on figure 1, the parallax barrier 30 comprises a plate, arranged parallel to the surface of the display panel, and at a distance noted f from the surface of the display panel 20. The plate comprises a plurality of pinholes 31, or microholes, arranged with a pitch, noted Δx, along axis X. Generally, the pitch p of the pixels is much smaller than the pitch Δx of the pinhole array. In addition, the light field display 60 presents a pitch Δy along axis Y (not shown in figure 1). The LFD 60 generates a 3D light field 69 everywhere in a semi-space above the micro-holes 31. Figure 1 shows an image plane 50 parallel to the light field display device 60, at a distance 1 from the parallax barrier 30. The image plane 50 is used for 4D representation of a light field beam. Each light field beam may be represented by a set of four coordinates (x, y, u, v) wherein (x,y) represent the coordinates in the plane of the micro-holes and (u,v) the coordinates in the plane 50 at distance 1. A minimum angle Δθ between two consecutive light rays generated by adjacent pixels passing through the same pinhole gives the angular resolution of the macropixel 222. A maximum aperture of the light field 69 has an angle θ_{MAX} which depends on the pinhole pitch Δx and on the distance f.

However, other kinds of parallax barrier 30 can be used instead of the pinholes array and thus be simulated according to the method of the current disclosure. For example, the LF may be projected directly in the pupil of the eye, by creating virtual pinholes. Each virtual pinhole is associated with a specific source switched on individually. For each virtual pinhole all the directions are driven by a spatial light modulator (SLM).

Figure 2 shows a side view of another known light field display device 60 comprising a high-resolution display panel 20 combined with a lenslet array 40. The lenslet array 40 comprises an array of microlenses 41, 42, 43... placed in near proximity of the display panel 20. For instance, the lenslet array 40 is arranged on top of the display panel 20. The pitch between microlenses is noted Δx, along axis X. As a function of the pixel 21 which is addressed, the light field passing through the microlens 44 has a specific vector u. As a result, the LFD 60 generates a 3D light field in an image plane 50 parallel to the LFD, at a distance 1 from the lenslet array 40. The light field generated depends on the position of the pixel which is activated.

Of course, the light field display devices described above can operate in a similar way along direction Y, with a 2D array of pixels and a 2D pinholes array, or respectively, a 2D grid of reflecting surfaces, or a 2D lenslet array, for generating a 4D light field.

The light field display device 60 enables to control the direction of rays coming from one point of an image. Thus, a light field display device 60 enables to control the position and direction of each ray forming the light field without using additional lenses of variable focusing distance. As a result, we can display virtually an image at multiple distances using only a light field display device at a constant distance from the eyes of a subject. More precisely, the light field display device 60 enables to form an image at a controlled distance from the light field display device.

However, parallax barrier LFDs or lenslet array LFDs generally impose a trade-off between spatial and angular resolution.

The properties of light fields may be used for correcting a refractive error of a subject's eye.

Let us consider a light field of parallel beams generated using a light field display device of any type as mentioned above. The accommodation of the subject's eye is relaxed. A visual stimulus is displayed at infinity. In case the subject's eye is emmetropic, the image (for example of a point) formed by the emmetropic eye, through the cornea, the pupil and the crystalline lens, is focused on the subject's retina. Thus, the retinal image of a source point is focused on a point on the retina and perceived sharply. In contrast, in case the subject's eye is relaxed, but the eye is affected by myopia, the image (for example of a point) formed by the myopic eye (or shortsighted eye) is focused on a point in front of the subject's retina. Thus, for a myopic eye receiving a light field of parallel beams, the retinal image of the point extends over an area and is perceived as blurred. For example, a light field display device may be used to generate a light field of deviated beams, so as to compensate for the aberration of the myopic eye. Then, when the myopic eye is relaxed, the image (for example of a point) formed by the myopic eye is focused on the subject's retina 19. Thus, the retinal image of the point is perceived sharply by the myopic subject.

Similarly, for a subject having a hypermetropic eye, which is relaxed, the image of a point formed using a light field with parallel beams is focused behind the subject's retina. Knowing the hypermetrope power value, it is possible to generate a light field of convergent beams so as to compensate for the aberration of the hypermetropic eye. Then, when the hypermetropic eye is relaxed, the image (for example of a point) of the LF with convergent beams formed by the hypermetropic eye is focused on the subject's retina. Thus, the retinal image of the point is perceived sharply by the hypermetrope subject.

More generally, knowing the refractive error of the eye, it is also possible to generate a light field which will be seen in focus by the eye. Using a light field display device, it is possible to generate a light field corresponding to different ocular aberrations along different directions, for example along X and Y directions. It is thus possible to compensate not only for spherical errors but also astigmatism, knowing the direction of the axis and the amount of astigmatism error (or cylinder) of the eye.

This could also include high order aberrations (HOA). The generated light field is thus optimized to form a sharp image on the retina. a

Thus, the light field display properties allow defining positioning and direction of rays on its surfaces. These properties are used in the method according to the current disclosure.

The properties of light fields are herein used to simulate an ophthalmic corrective lens with a light field display in order to correctly display to a user a scene seen through the corrective lens by using a real light field display.

### Process

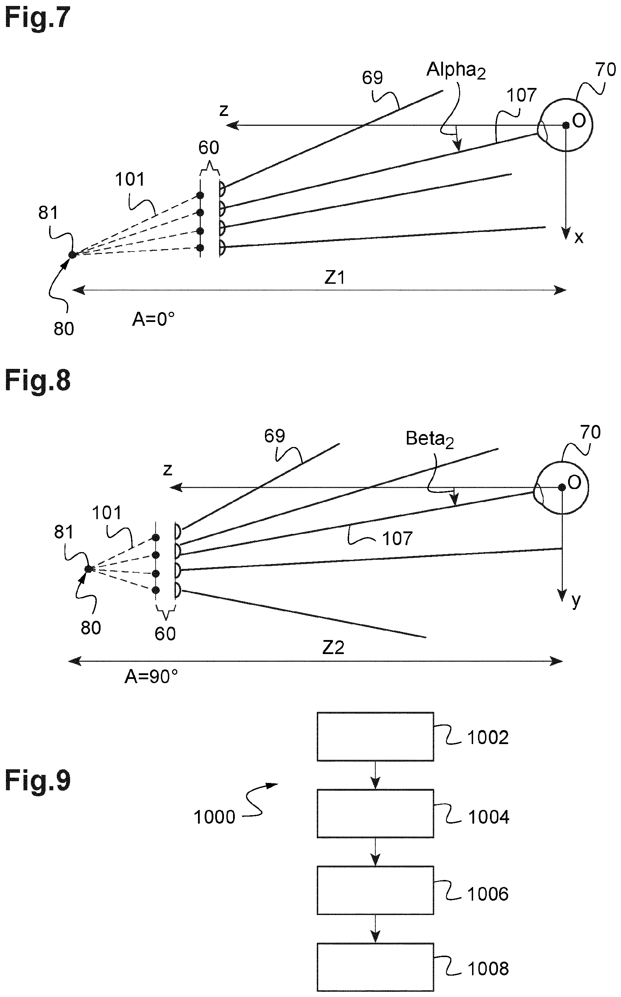

Figure 9 illustrates a block diagram of a method 1000 for simulating an ophthalmic lens on an eye 70 of a subject viewing a virtual three-dimensions scene 80 using a light field display 60, according to an exemplary embodiment.

Virtual means something that is simulated and/or calculated.

By virtual three-dimensions (3D) scene, it is meant a representation of at least one object or a plurality of objects for which the 3D coordinates in space and the colour are known. For example, a virtual 3D scene 80 represents a single object 81 or a plurality of objects 81. In addition, a virtual 3D scene may be an image comprising at least one object or a plurality of objects. In an embodiment, the virtual three-dimensions scene 80 is divided into a plurality of points, wherein each point of the scene 80 has three-dimensions positions in space, preferably relative to the eye 70 of the subject 71. In another embodiment, the virtual three-dimensions scene 80 is a virtual point.

The light field display 60 according to the present disclosure comprises a light field window 65.

The light field window 65 is the surface of emission of the light field display 60. It is analogous to a standard window through which one would look at a 3D scene. In the following example, the light field window 65 corresponds to an active area of the light field display 60 that is used in the method according to the current disclosure. Generally, the light field window 65 comprises a surface of emission arranged to generate a light field 69 towards the eye 70 of the subject. The light field display 60 comprises or is linked to a calculation unit configured to carry out the steps of the method 1000 according to the current disclosure.

By a calculation unit, it is meant a computer or a processor arranged to carry out instructions to carry out the steps of the method according to the current disclosure.

The method 1000 comprises a step of receiving 1002 a set of input parameters comprising scene parameters of the virtual three-dimensions scene 80. Thus, generally, the scene parameters are already known. For example, the scene parameters comprise an image as input parameter or a set of 3D coordinates of points representing one or more 3D objects.

Optionally, the set of input parameters comprises other parameters, such as light field display parameters and subject parameters. For example, the subject parameters comprise at least pupil diameter of the eye 70 and a pupil position of the eye 70 preferably relative to the light field display 60. The light field display parameters are generally determined as a function of the technical feature of the light field display 60. For example, the light field display parameters comprise dimensions of the light field display 60 and/or of the light field window 65. In addition, the light field display parameters comprise a number of pixels 64 comprised in the light field display 60, a spatial density of the pixels and/or a pixel size of these pixels.

In addition, the method 1000 comprises a step of calculating 1004, by ray tracing, a virtual ray 100 between a point of the virtual three-dimensions scene 80 and a point of a plane 72 of the pupil of the eye 70, the virtual ray 100 passing through a virtual lens 90. According to method 1000, the virtual ray 100 is defined on the basis of a model providing a deviation angle of the virtual ray 100 through the virtual lens 90, the deviation angle depending on at least one incidence angle and position of the virtual ray 100 on the virtual lens 90. Then, the method 1000 is configured to repeat the step of calculating 1004 by ray tracing for a plurality of virtual rays 101 passing through the virtual lens 90 and joining couples of one point of the virtual three-dimensions scene 80 and another point of the eye 70 pupil plane 72, so as to scan spatially and angularly the light field window 65.

Preferably, the step of calculating 1004 is further based on the sets of input parameters, such as, for example light field display parameters, subject parameters and scene parameters.

Then, the method 1000 comprises a step of determining 1006 a light field 69 representing a modified virtual three-dimensions scene 140 based on the scene parameters and on the deviation angle associated to each virtual ray of the plurality of virtual rays. The determination carried out by the step of determining 1006 the light field 69 representing a modified virtual three-dimensions scene 140, 83 is also based on an angular and spatial sampling of the light field display 60.

By light field 69, as explained in the definition part, it is meant an amount of light flowing in every direction through every point in space. According to the method 1000, the light field 69 determined in the step of determining 1006 is preferably calculated to reach the eye 70 of the subject, and, in particular, the pupil of the eye considered.

The method 1000 comprises a step of generating 1008 the light field 60 representing the modified virtual three-dimensions scene 140, 83 on the light field window 65 towards the eye 70 of the subject 71. In the step of generating 1008, the light field 69 generated is real. It means that it is arranged to really reach the eye 70 of the subject 71.

By modified virtual three-dimension scene 140, 83, it is meant the image of the virtual three-dimensions scene 80, which is displayed to the subject 71.

Although the method 1000 is disclosed for simulating an ophthalmic lens on an eye 70 of a subject 71, the method 1000 may be adapted for simulating an other ophthalmic lens 90 on an other eye 70 of the subject 71. In this way, the method 1000 in this embodiment comprises steps of calculating 1004, determining 1006 and generating 1008 for simulating the other ophthalmic lens 90 for the other eye 70 of the subject 71. In addition, the subject parameters in the step of receiving 1002 comprise other parameters among a pupil diameter of the other eye 70 and/or a pupil position of the other eye 70 relative to the light field display 60 allowing, and/or interpupillary distance between the two eyes 70 to generate an optimized light field 69.

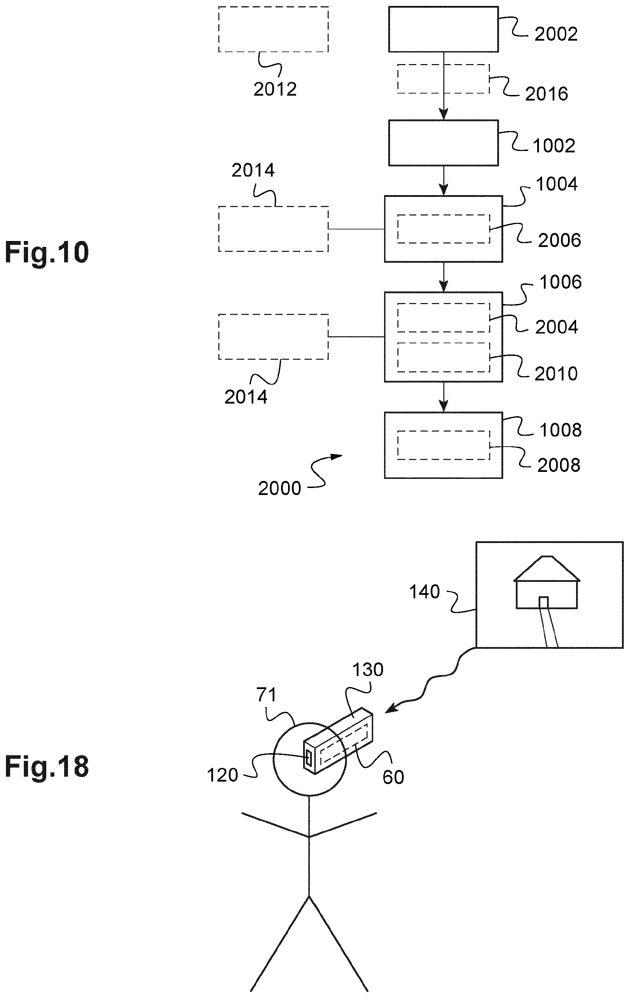

Figure 10 illustrates a block diagram of a method 2000 for simulating an ophthalmic lens on an eye 70 of a subject 71 viewing a virtual three-dimensions scene 80 using a light field display 60. The method 2000 disclosed in figure 10 comprises all the steps disclosed in the method 1000 illustrated in figure 9. Thus, only the differences between figure 9 and figure 10 will be described.

When the scene parameters are not known, the method 2000 comprises, before the step of receiving 1002, a step of acquiring 2002 a three-dimensions image of the virtual three-dimensions scene using a three-dimensions capturing device to extract the scene parameters, said scene parameters comprising geometric and/or photometric properties of the virtual three-dimensions scene 80. In addition, even if the scene parameters are known, the step of acquiring 2002 may allow to verify or complete the scene parameters allowing to improve the realism of the simulation of the method according to the current disclosure. In another embodiment, as for the method 1000, the scene can be artificial. By artificial, it is meant a scene extracted from an image. The image can be an acquired image or handmade image such as a drawing, or an image obtained with a software by artificial intelligence, etc.

Then, the method 2000 optionally comprises a preliminary step 2012 configured to determine a distance between the light field display 60 and the eye 70 of the subject 71. For example, the preliminary step 2012 comprises a step of acquiring an image of the eye 70 or both eyes of the subject 71 using an image capturing device, said image capturing device being arranged on the light field display 60 or on a frame of the light field display 60. Then, the preliminary step 2012 comprises a step of deducing from the acquired image, the distance between the eye or between both eyes 70 of the subject 71 and the light field display 60. This deduction can be realized, for example, knowing the size of a particular feature in the image, for example, the inter-pupillary distance, or knowing the focus used to acquire the image. The distance is set to the distance measured between the light field display 60 and the eye 70 of the subject 71. This preliminary step 2012 optionally determines an interpupillary distance between both eyes 70 of the subject 71. In this way, the method 2000 according to the current disclosure acquires real data of the subject 70, which may be used to verify or complete the subject parameters.

In addition, the step of determining 1006 comprises a step of applying 2004 geometric parameters of the virtual three-dimensions scene 80, and in particular, the photometric properties of the virtual three-dimensions scene 80 allowing to consider other properties of the virtual three-dimensions scene 80, so as to improve the rendering of the virtual three-dimensions scene 80 to the subject 71.

The geometric parameters comprise three-dimensions cartography of the virtual three-dimensions scene 80 in order to obtain a real three-dimensions position of the at least one object of the virtual three-dimensions scene 80 or to obtain all the real three-dimensions positions of the objects 81 and elements included in the virtual three-dimensions scene 80. By position, it is meant the coordinates of at least one object or the coordinates of all the points of the object. The three-dimensions positions are preferably expressed relative to the eye 70 of the subject 71 allowing to improve the robustness and the accuracy of the steps of the method 2000 according to the current disclosure.

For instance, the image capturing device comprises or may be a plenoptic camera.

By photometric properties, it is meant the radiant energy, and/or the light reflection model. Thus, the photometric properties comprise the radiant energy of the at least one object 81 of the virtual three-dimensions scene 80, preferably the radiant energy of all the objects 81 and/or elements included in the virtual three-dimensions scene 80. In addition, the photometric properties comprise the model of light reflection model of the at least one object 81 of the virtual three-dimensions scene 80, preferably a model of light reflection of all the objects 81 or elements included in the virtual three-dimensions scene 80. This radiant energy can also comprise the spectrum.

Optionally, the step of calculating 1004 and/or, respectively the step of generating 1008, of the method 2000 comprises a step of selecting 2006, respectively 2008 a subset of the plurality of virtual, respectively real, rays passing through the pupil of the eye 70 of the subject 71.

In this example, the step of calculating 1004 comprises a step of selecting 2006 a subset of the plurality of virtual rays passing through the pupil of the eye 70 of the subject 71. Thus, it is meant that the step of calculating 1004 is configured to calculate only virtual rays that reach the eye 70 (i.e. virtual eye used in the ray tracing of the step of calculating 1004). Virtual rays calculated by ray tracing that do not reach the eye (virtual eye) of the subject are not calculated or not considered. Thus, the step of calculating 1004 is optimized because only virtual rays of interest are calculated in the step of calculating 1004. The step of calculating 1004 is less time-consuming, especially in case the scattering effect is taken into account. The method according to the current disclosure is thus optimized.

In this example, the step of generating 1008 comprises a step of selecting 2008 a subset of the plurality of rays passing through the pupil of the eye 70 of the subject 71. Thus, it is meant that the step of generating 1008 is only arranged and/or configured to generate a light field 69 based on a subset of rays that reach the eye (i.e. virtual eye) of the subject in the step of calculating 1004.

The step of generating 1008 is optimized because the generated light field 69 is only based on rays liable to reach the eye 70 (real eye) of the subject 71. The step of generating 1008 is thus faster, more robust and accurate. The method 2000 according to the current disclosure is thus optimized.

Optionally, the step of determining 1006 comprises a step of representing 2010 the modified virtual three-dimensions scene 140, 83 further comprising applying a convolution or image deformation, adapted for simulating blur, distortion, diffraction or chromatism. The EP patent application reference EP2020905B1 and the PCT application reference WO2009077617A1 explain such image deformation or distortion.

In another embodiment, this step of representing may be used to test the perception of the subject 71 after carrying out the step of representing 2010. The refraction parameters of the eye 70 of the subject 71 may be used, so as to improve the refraction correction of the subject 71.

Optionally, the method 2000 comprises a step of simulating 2014 the light field display 69 relative to the eye 70 of the subject 71 and relative to the virtual three-dimensions scene 80. For example, the step of simulating 2014 corresponds to a step that displays a virtual light field display 60 between a virtual eye 70 of a subject 71 and a virtual three-dimensions scene 80. In this way, the steps of calculating 1004 and determining 1006 are carried out in real time. The method 2000 according to the current disclosure is thus faster because only rays entering the eye 70 are selected.

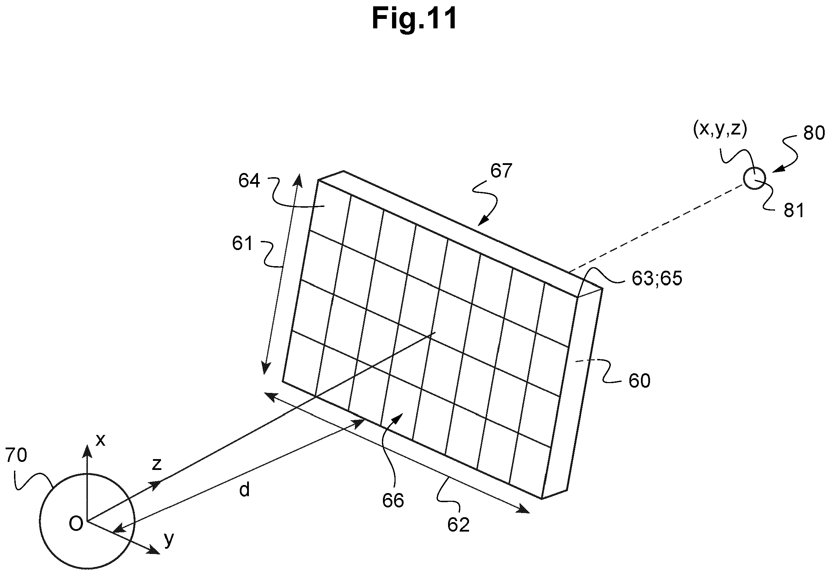

Figure 11 illustrates a light field display 60 positioned in front of an eye 70 of a subject 71 so as to display the virtual three-dimensions scene 80. The light field display 60 represented in figure 11 is a general one. The figures 1 and 2 illustrate specific realization of the light field display 60, the virtual three-dimensions scene 80 is also called scene 80.

Generally, the light field display 60 comprises dimensions between 3 to 147 millimeters (mm). In this example, the surface of the light field display 60 is arranged to extend spatially over a surface area of at least 3 mm in a horizontal plane and 3 mm in a vertical plane. In this example the light field display 60 is arranged to extend along a first direction 61 and along a second direction 62. Optionally, the first and second directions 61, 62 are perpendicular. The light field display 60 is, for example, of a minimum of 3 millimeters (mm) according the first direction 61 and a minimum of 3 millimeters according the second direction 62. In another embodiment, the light field display 60 can be of 7 mm x 7 mm or 8 mm x 8 mm. A light field display 60 of this size can be used for a head mounted display (wearable headset) that is more immersive than standalone screens. In addition, the light field display 60 presents a spatial resolution of at least 0,5dot/mm along the first and/or the second directions 61, 62. For example for a light field display 60 of 8 mm by 8 mm, the spatial resolution is of 1dot/mm.

The light field display 60 illustrated in figure 11 is made up of an array of pixels 63, which is arranged to project and generate at least one light field to one eye 70 of the subject. In this example, the array of pixels 63 is a matrix of pixels 64 (the pixels 64 are also numbered 21, 22 on figures 1 and 2), said matrix of pixels 64 comprising a plurality of lines and columns. The matrix of pixels 64 extends spatially according to the first 61 and second 62 directions. The array of pixels 63 defines the surface of emission of the light field display 60.

The light field display 60 has a light field window 65. Generally, the light field window 65 corresponds to the surface of emission of the light field display 60, therefore the light field window 65 and the light field display 60 have the same size.

The light field window 65 may be illustrated on the first side 66 and the second side 67. According to the current disclosure, it is to be understood that the light field window 65 is an active surface, which is activated and used in the steps of the method 1000, 2000 according to the current disclosure.

In this example, the light field 69 content corresponds to a field of view of at least 40 degrees compared to the gaze axis of the eye 70, in a horizontal plane and 40 degrees in a vertical plane and/or an angular resolution of less than 5 degrees allowing to display small details with high resolution. For example, the angular resolution is of a few minutes of arc for example 5 minutes.

By angular resolution, it is meant an angular pitch of emission that may be carried out by the light field display 60, illustrated in figure 1 and in figure 2 by the angle Δθ.

The light field display 60 is positioned relative to the eye 70 of the subject 71. Particularly, a referential coordinate system is positioned on the eye 70 of the subject. These features enable the steps of the method 1000, 2000 to be easy to implement while being accurate.

A distance d separates the light field display 60 from the eye 70 of the subject. Preferably, the distance d is known. The distance d is generally part of the light field parameters in order to position the light field display 60 relative to the eye 70 of the subject 71.

The distance d is generally comprised between 5 and 85 millimeters, for virtual reality or augmented reality headset application designed for short distance or the distance d may be comprised between 100 millimeters to 1000 millimeters for smartphone or tablets applications designed between short and medium distance, or the distance d may be comprised between 2 meters to 5 meters for TV-like use designed for far distance. In another example, the distance d is of a few millimeters maximum, and as close to 0 as possible. Indeed, the surface of emission in this case is note delimited by a mechanical frame. In another embodiment, the distance d is acquired in the preliminary step 2012, for example with an image capturing device arranged on the light field display 60. The distance d corresponds to the distance measured between the light field display 60 and the eyes 70 of the subject 71 allowing to provide the real condition of view to the subject 71. This preliminary step 2012 may optionally determine an interpupillary distance between both eyes 70 of the subject 71 and the pupil diameter of each eye 70 of the subject 71. The image capturing device may be a camera or a time-of-flight sensor.

In this example, the horizontal plane (including the second direction 62) of the light field display 60 also includes the axis z of the referential coordinate system arranged relatively to the eye 70 of the subject 71, and centered on the pupil center, noted 0 or center of rotation, noted CRO of the eye 70. The center of rotation (CRO) is preferred since not dependent of the convergence of the eyes 70 when looking at near objects The first direction 61 and the second direction 62 are here perpendicular to the axis z.

The scene 80 illustrated on figure 11 is virtual. In this example, the scene 80 comprises one object 81 (i.e. virtual object) having a three-dimensions position expressed relative to the eye 70 of the subject 71. For example, the object 81 has three-dimensions coordinates noted (x, y, z). It means all the points of the object have known coordinates.

As shown in figure 11, the (0, x, y, z) referential coordinate system is on the eye 70 of the subject 71. The referential coordinate system comprises three axes, respectively noted x, y, z. The z axis passes through the pupil center of the eye 70. In figure 11, the center of rotation of the eye is illustrated by the origin O of the referential coordinate system.

Although only one eye 70 is illustrated at figure 11, the light field display 60, preferably the array of pixels 63 of the light field display 60, is arranged to project or generate an other light field 69 to an other eye 70 (not illustrated at figure 11) of the subject 71. Thus, the light field display 60 is arranged to generate a first light field towards the first eye 70 of the subject 71 and a second light field 69 towards a second eye 70 of the subject 71. The first light field 69 and the second light field 69 can be generated simultaneously or alternatively. In this way, the method 1000 or 2000 is configured to simulate an other light field 69 afront the other eye 70 of the subject 71. This method allows to apply the convergence of the vision of the subject 71. The convergence is automatically applied by the ray tracing when the position of each eye 70 relative to the object 71 is taken into account. Thus, the pupillary distance and the difference of point of view between the 2 eyes is taken into account. The subject 71 will then converge to merge the 2 images has he/she would made on a real object. For the demonstration and evaluation of an ophthalmic correction in far vision, the scene 80 or the at least one object 81 of the scene 80 is preferably arranged close to the infinity to reduce accommodation, independently of the physical position of the light field display 60.In another embodiment, figure 11 illustrates the step of simulating 2014 (optional step) used in the step of calculating 1004, wherein the light field display 60, the eye 70 and the scene 80 are all virtual. These parameters also comprise the angular resolution Δθ and a maximum angle θₘₐₓ referring to the maximum angle θₘₐₓ of emission of rays passing through the same point 46 of the light field window 65.

For example, the light field display parameters comprise at least the number of pixels, the size of the pixel, spatial density of the pixels and/or the dimensions of the light field display 60 and/or the light field window 65 and the distance d separating the light field display 60 from the eye 70 of the subject.

The light field display parameters are used as input for at least the steps of calculating 1004 and determining 1006. For example, the light field window 65 is function of the light field display parameters. In addition, the scene parameters comprise the three-dimensions positions of the object 81 of the scene 80, for example the coordinate (x, y, z) of the object 81.

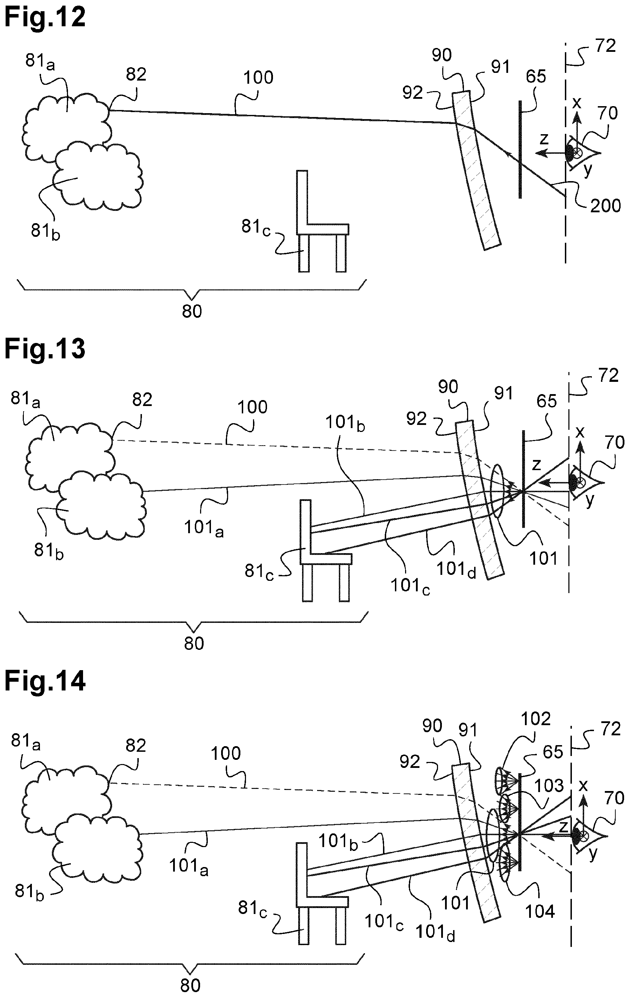

Figures 12, 13, 14 represent schematically an example of embodiment of the steps of calculating 1004 and determining 1006.

The figures 12, 13, 14 show virtual rays of one view for clarity's sake.

Figure 12 illustrates a scene 80, a light field window 65 positioned in front of the eye 70 and a virtual lens 90 positioned between the scene 80 and the light field window 65. In this example, the scene 80, the light field window 65, the eye 70 and the virtual lens 90 are simulated, for example, by a lens design software. Thus, they are not real.

It is noted that the light field window 65 shown in figures 11 to 14 is a schematic representation of a side view of the light field window 65 belonging to the light field display 60 illustrated for example in figures 1 and 11. However other types of light field display 60 can be used to realize the different embodiments.

The virtual lens 90 is represented by a model, which is typically the model of a real ophthalmic corrective lens to be worn by the subject. The virtual lens 90 comprises a first diopter 91, oriented towards the eye 70 and a second diopter 92 oriented towards the scene 80. Each diopter 91, 92 enables to deviate a ray, i.e. virtual ray 100, according to an angle of deviation. Thus, each virtual ray 100, 101 that passes through the virtual lens 90 is deviated by the first and the second diopters 91, 92.

By model of the virtual lens 90, it is meant the lens design and the optical properties of the virtual lens 90. Thus, it is to be understood that the virtual lens 90 refers to the simulated physical means, whereas the model refers to optical properties of the associated virtual lens.

For example, the model of the virtual lens 90 shown in figure 12 comprises a shape of the ophthalmic corrective lens (i.e. real lens) and a positioning of the ophthalmic corrective lens in relation to the eye 70 of the subject 71 and also the optical properties of the material (for example refractive index and chromatic dispersion properties). The positioning of the virtual lens 90 is thus relative to the eye 70. Preferably, the shape of the ophthalmic corrective lens comprises the shape of the first and second diopters 91, 92. For example, the shape of the diopters 91, 92 may be convex, concave, planar, spherical, aspherical, cylindrical, toric, progressive, etc...

The model of the virtual lens 90 is determined before the step of calculation 1004. The model is known, for example it corresponds to the lens design of the ophthalmic corrective lens of the eye 70 of the subject 71. Generally, this lens design model comprises at least a geometry of front and back surfaces of the lens, a refraction index, and a position of this lens in space. Alternatively, the model 90 is determined by calculations. In that case, the model is determined on the basis of geometrical optics calculations and/or wave optics calculations.

The model of the virtual lens 90 illustrated in figures 12-14 comprises distortion model of the virtual lens 90. Generally, this model of distortion comprises at least a sphere parameter and astigmatism power model of the virtual lens comprising at least a cylinder and an axis parameter or J0 and J45 parameters. This model allows to take into account all the refraction parameters and the dioptric effects of a real lens (i.e. the ophthalmic corrective lens), to be worn by the subject 71.

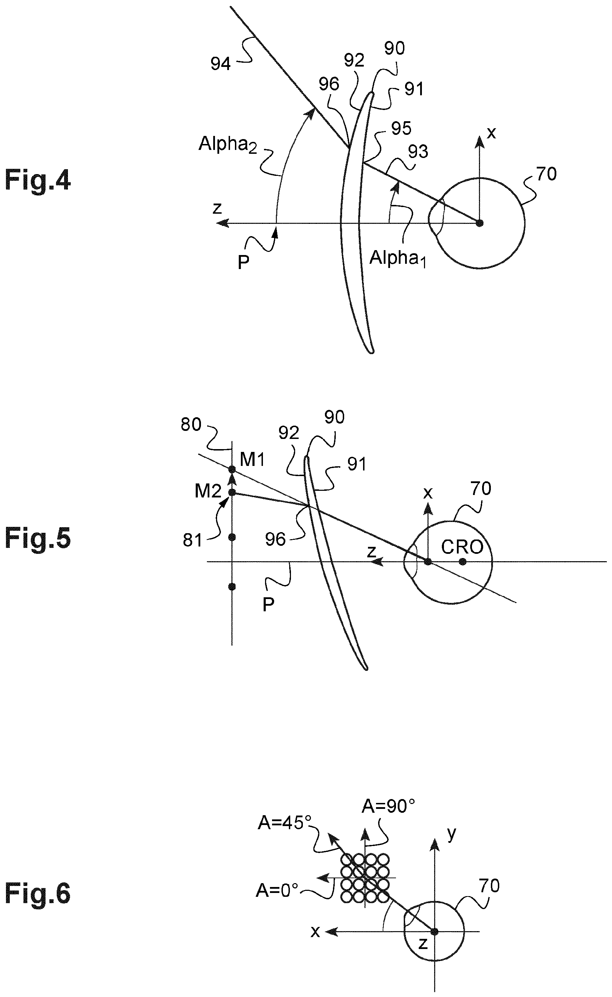

For example, figures 3, 4 show calculations to determine the model of distortion, spherical power model and the astigmatism power model. These models are calculated in an optional step of calculating 2016 carried out before the calculation step 1004.

Figure 3, 4 show an exemplary distortion model built using a simulation method. In this example, a virtual lens 90 is illustrated relative to the eye 70 of the subject 71. To get the distortion model of the virtual lens 90, the calculator generates a set of rays (virtual rays) emerging from the pupil of the eye 70 and calculates deviations angles at an exit of the virtual lens 90. **In** this calculation, each ray propagates along a different direction and each ray is deviated by the first diopter 91 and the second diopter 92 of the virtual lens 90. The virtual lens 90 presents an optical axis, noted P, colinear to the z axis of the referential coordinate system. In this example, only one ray is shown. An initial ray 93 starts from the eye 70 of the subject 71, at a first end, and reaches the first diopter 91 at a second end. The initial ray touches the first diopter 91 at a point of the virtual lens 90 noted 95. The initial ray 93 comes from the center of the eye 70 and is deviated by refraction thought the virtual lens 90 to form a deviated ray 94 at the exit of the virtual lens 90 on the second diopter 92. The deviated ray 94 intersects the second diopter 92 at a point noted 96. The initial ray 93 coming from the eye 70 presents, on the point 95 of the first diopter 91, an angular position defined with initial angles called noted alpha₁ and beta₁. The initial angles are expressed relative to the referential coordinate system (o, x, y, z) of the eye 70. The angle alpha is defined relatively to the axis z in the plane (o, x, z). The angle beta is defined relatively to the axis z in the plane (o, y, z). At the point 96 of the second diopter 92, the deviated ray 94 is at an angular position expressed according to deviation angles called alpha₂, beta₂. A deviation between the initial ray 93 and the deviated ray 94 is calculated with typical refraction law, such as Snell-Descartes law. Thus, using the lens geometry and ray tracing method, the angles of deviations are obtained. For example, the distortion model of the virtual lens 90 for an incident ray i may be expressed with the formula: ${P}_{alpha} \left({alpha}_{1 , i}, {beta}_{1 , i}\right) = {alpha}_{2 , i ′} - {alpha}_{1 , i}$ and${P}_{beta} \left({alpha}_{1 , i}, {beta}_{1 , i}\right) = {beta}_{2 , i ′} - {beta}_{1 , i}$ where i corresponds to index of the initial ray 93 and i' corresponds to the index of the deviated ray 94, and Pₐₗₚₕₐ, and P_{beta} represent the angular deviation according to alpha and beta introduced by the virtual lens 90 along the gaze direction (alpha, beta)and i represents the index of the initial ray.

In this example, the virtual lens 90 has a thickness, which is not constant, it varies depending on the position on the virtual lens 90. Thus, the virtual lens 90 shown presents an optical power that varies over the field of the virtual lens 90. To obtain the model of deviation of the virtual lens 90, the virtual lens 90 is scanned spatially and angularly over its whole surface area in order to determine the angles of deviations at each point of the virtual lens 90, thus for each point 95 of the first diopter 91 and for each point of the second diopter 92. Thus, a scan of the angles of deviation expressed according alpha₂, beta₂ is carried out.

If the step of calculating 1004 is carried out without any virtual lens 90, the light field display 60 provides a modified virtual three-dimensions scene 80 with the object 81 in the three-dimensions space without any deformation. Thus, to take into account the deviation model, the positions of the object 81 in the scene 80 (virtual scene) are moved so that the eye 70 sees the object 81 according to angles of view noted alpha₂, _{object} and beta_{2,object} with: ${alpha}_{2 , object} = {alpha}_{1 , object} - {P}_{alpha} \left({alpha}_{1 , object}, {beta}_{1 , object}\right)$ and ${beta}_{2 , object} = {beta}_{1 , object} - {P}_{beta} \left({alpha}_{1 , object}, {beta}_{1 , object}\right) .$

Figure 5 shows an example of an initial object point M1 seen through the virtual lens 90 at the distorted position M2 calculated by ray tracing going through the pupil of the eye 70 of the subject 71. In that case the light field, which would be calculated for point M1 is applied in point M2 to simulate the distortion model of the virtual lens 90. The same process is applied for the different colors to simulate chromatism effects. The distortion calculations may be implementing in the same manner as distortion calculations provide in the disclosure of document EP 2 020 905.

In addition, in an embodiment, the virtual lens 90 presents an attenuation factor. It means that an intensity or a radiance energy of a virtual ray passing through the virtual lens 90 is attenuated by the virtual lens 90. The attenuation depends on the attenuation factor of the virtual lens 90. Consequently, the luminous intensity of the object M1 in the virtual scene 80 is attenuated by the attenuation factor of the virtual lens 90. The attenuation depends on the transmission of the virtual lens 90 at the position where the virtual rays emitted from the object M1 passes through the virtual lens 90, for example at le level of the point numbered 96 on the virtual lens 90.

In another embodiment, the virtual lens 90 is further characterized by a transmission factor and scattering function depending on the position where the virtual ray passes through the virtual lens alpha_{1,i}, beta_{1,i}, and the wavelength of the virtual ray (or its spectral characteristic).

If the scattering function of the virtual lens 90 is constant with respect to angles alpha_{1,i}, beta_{1,i}, the transmission of the virtual lens 90 is uniform. The transmission could be due to absorption or reflection, with a wavelength dependence.

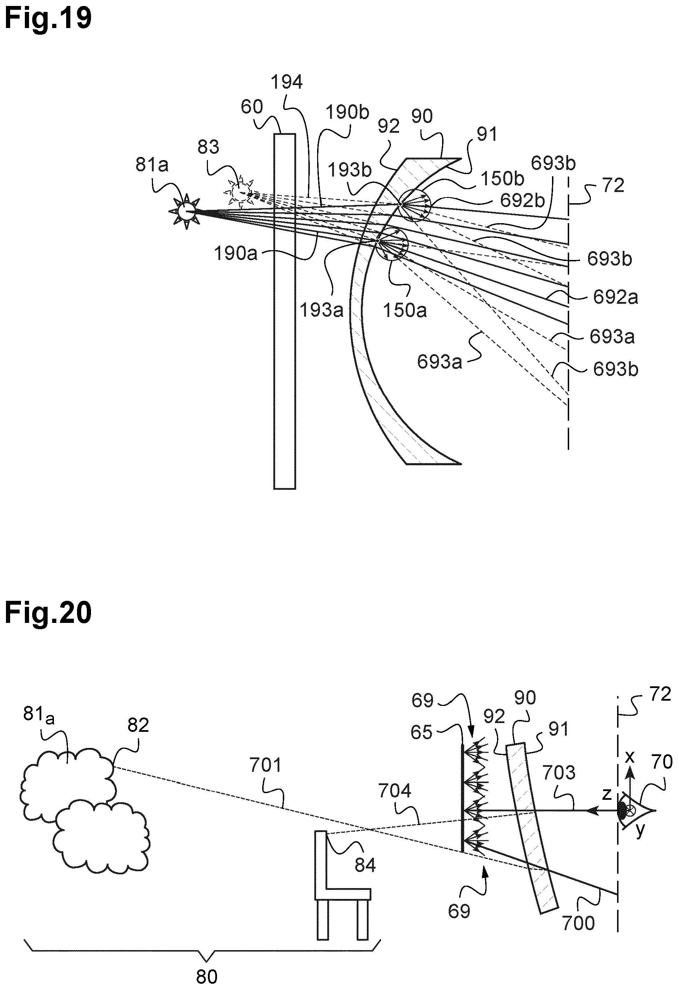

The scattering function in the present disclosure could be modelled by the bidirectional transmittance distribution function, noted BTDF and also known as Bidirectional Scattering Function and numbered as 150. The scattering function can be implemented in the step of calculating 1004. The BTDF to be simulated could comprise:
- the scattering model known as geometric scattering model that can be used in case of rough surfaces with defects larger than the wavelength;
- the Mie scattering, due to particles about same size of wavelength;
the Rayleigh scattering with particles smaller than wavelength. The scattering function in the present disclosure is also considered in the step of generating 1008 (see figure 19).

The simulation of the BTDF in the step of calculating 1004 can be time consuming. To limit the calculation time, the method of calculating 1004 can be faster when a simple object 81 from the virtual scene 80 is close to a flat image. The simulation of scattering could be obtained, for example, by an angular convolution. It is more efficient to take into account only the virtual rays passing through the pupil of the eye, and not the rays stopped by the iris, sclera, or other non-transparent medium crossed before the retina.

To improve the calculation time of the step of calculating 1004, the method of the present disclosure can implement the BTDF as the method disclosed in the following publication "Image-based Rendering of Spatially-Varying BSDF", 2010 by Hongsong Li and Fengxia Li.

One benefit of the light field display 60 is that it is possible to simulate the power variation of the lens as it would be perceived by user, and not using a blur function, playing on a position defined between the virtual object and the eye of the subject. This position is noted zᵢ in the figures 6, 7, 8. The object 81 is expressed with the angular position given by the angles of deviations alpha₂, beta₂. For example, the coordinates of the object may be noted object(alpha₂, beta₂, z₁) after a distortion correction. Then, the distance z1 between the object and the eye depends on the refraction parameters of the eye 70 of the subject 71.

For example, when only the spherical power model, noted Sphere, of the virtual lens 90 is considered, the distance z₁ between the object and the eye of the subject now may be changed according to a distance noted z₂ as the following formula: $\frac{1}{{z}_{2}} = \frac{1}{{z}_{1}} - Sphere \left({alpha}_{2}-{beta}_{2}\right)$

And when the spherical power model and the astigmatism power model of the virtual lens 90 are considered, the distance between the object of the scene 80 and the eye 70 of the subject 71 depends on the axis and the cylinder of the parameters of astigmatism. The axis and the cylinder are noted Axe(alpha₂, beta₂ and the Asti(alpha₂, beta₂). The distance z₁ between the object 81 of the scene and the eye 70 of the subject 71 is now changed according to a distance z₂ as the following formula: $\frac{1}{{z}_{2}} = \frac{1}{{z}_{1}} - \left(Sphere\left({alpha}_{2}-{beta}_{2}\right)\right) + Asti \left({alpha}_{2}-{beta}_{2}\right) ∗ cos \left(2\left(\begin{matrix}A - \\ Axe \left({alpha}_{2}-{beta}_{2}\right)\end{matrix}\right)\right) with A being a direction of a meridian line .$

Figures 6, 7, 8 show an example of embodiment to determine the distance between the virtual object 81 and the eye 70 of the subject 71 considering the astigmatism power model of the virtual lens 90. The properties of the light field display are used. In this example, light fields generated towards the eye 70 of the subject take into account the spherical power and the astigmatism power models of the virtual lens 90 using the lenslet of the light field display 60 shown in figure 2. In figures 6, 7, 8, an axis of astigmatism A of ninety degrees is considered (see figure 6). Rays 107 of the light field 69 that reach the eye 70 of the subject 71 have an angular position expressed according to the deviation angles alpha₂, beta₂. The distance between the virtual object 81 and the eye 70 of the subject 71 depends on each focus plane. For example, according to the focus plane z, x, the distance between the virtual object 81 and the eye 70 of the subject is noted z₁ (figure 7), whereas the distance between the virtual object 81 and the eye 70 of the subject 71 according to the plane of view z, y is noted z₂ (see figure 8). It means according to the plane z, x, the lenses of the light field display 60 simulate a point at a distance z₁ (i.e. maximal distance), whereas the lenses of the light field display 60 simulate a point at a distance z₂ (i.e. maximal distance) according the plane z, y. If an axis A of forty-five degrees is considered, thus the distance between the virtual object 81 and the light field display 60 is expressed as z₃ = (z₁+z₂)/2 and it varies according to cos(2A) rule.

For each object 81 or point of object of the scene 80, this point, with some approximations, has a proximity P_{obj} that is equal to: ${P}_{obj} = \frac{1}{z} + {P}_{corr} + {P}_{eye}$

P_{obj} is independent of the distance d between the eye 70 and the light field display 60, with 1/z being the inverse of the object distance from the eye, P_{corr} being the resulting additional power of the ophthalmic correction at the point through which the object is seen, P_{eye} being the corrective power needed for the eye 70 to see a perfectly clear image at infinity or the desired distance.

For example, a light field generated for an object 81 with a proximity (i.e. distance z) of P_{obj} equal to the invert distance z (P_{obj} = 1/z), the eye 70 accommodating for this distance, z being independent of d even if the distance d is varying. Thus, the distance z of the virtual object 81 displayed is independent of the position of the light field display 60 (i.e. d). If the distance d is varying, for example if the light field display 60 is used on a smartphone, the properties of the light field display 60 may be recalculated in real time in order to maintain the distance z between the modified virtual object 81 and the eye 70 of the subject constant independently of the distance d.

The model may be modified based on parameters of real lenses worn by the subject. Thus, the light field is generated for a specific correction, for example for the sphere parameter (Sph) and the astigmatism parameter comprising a cylinder and an axis (Cyl and Axis), corresponding to the prescription part of the eye. In addition, the light field may be generated for a specific pupil diameter, a specific distance between the light field display 60 and the eye 70 of the subject, and other feature such as characteristics of the ophthalmic correction lens to demonstrate the specific optical properties and eventually high order aberration, and characteristics of the screen and the three-dimensions scene 80 comprising the distance between the eye 70 and the object 81.

In the example of figure 12 (and figures 11-15), the virtual lens 90, which is simulated, comprises a progressive power lens. Thus, the properties of complex lens may be simulated and replicated. In another embodiment, the virtual lens comprises a multifocal lens, a single vision lens, an eyeglass lens or a contact lens. The contact lens, as well as an Intra Ocular Lens (IOL) can be represented by a power profile model in the pupil plane.

In addition, the scene 80 illustrated in figure 12 comprises three objects called first, second and third objects 81a, 81b, 81c and arranged at different locations of the scene 80 and at different proximities.

In this example, a virtual ray 100 associated to a real ray 200 is illustrated in figure 12. The virtual ray 100 propagates from the virtual scene 80 to the light field window 65 and the real ray propagates from the light field window 65 to the eye 70 of the subject 71. To determine the value, for example, the gray value or the brightness level of the real ray 200 that should be generated in the step of generating 1008, the step of calculating 1004 is configured to calculate a virtual ray between 100 an eye pupil plane numbered 72 and the virtual scene 80. The virtual ray that reaches the scene 80 is associated to a color and/or a luminosity. The value of the real ray generated in the step of generating 1008 by the light field display 60 is attributed with the value of the color and/or luminosity of the point of the scene 80.

In this example, the virtual ray numbered 100 is calculated. The virtual ray 100 reaches a point of the scene numbered 82. The point of the scene has (x, y, z) coordinates and is associated to a color and/or a luminosity. The virtual ray 100 is deviated by the virtual lens 90. Then, the real ray 200 generated in the step of generating will have the color and/or the luminosity of the point 82.

After the calculation of the virtual ray 100 represented in figure 12, the step of calculating 1004 is configured to repeat the calculations by calculating a plurality of virtual rays 101.

In the example of the figure 13, different virtual rays 100, 101a, 101b, 101c, 101d are illustrated and form a plurality of virtual rays 101. In this example, the plurality of virtual rays 101 are configured to propagate from the eye pupil plane 72 to all the objects 81 of the scene 80. The plurality of rays 101 pass through a same point 46 of the light field window 65 but with different directions of emission (see figures 1 and 2 for the position of the point 46). The different directions of emission are each associated to a point of the light field window 65, which corresponds to a pixel 64. The different directions of emission that pass through the point 46 of the light field window 65 are associated to a number of adjacent pixels 64 that belong to a same line and/or a same column of the matrix of pixels 63. In this example, the number of adjacent pixels is given by a 2D pitch, which is in this example a pitch ΔyΔx, the light field window 65 being a two-dimensions light field window 65. The number of adjacent pixels given by a 2D pitch is associated to a macropixel 222.

In this example, the plurality of virtual rays 101 here comprises virtual rays respectively noted 101a, 101b, 101c, 101d. The plurality of virtual rays 101 reaches the scene 80 at points that differ from the point reached by the virtual ray 100 in the scene 80. For example, the virtual ray 101a of the plurality of virtual rays 101 reaches a point of the second object 81b, the virtual ray 101b of the plurality of virtual rays 101 reaches a point of the third object 81c, the virtual ray 101c of the plurality of virtual rays 101 reaches another point of the third object 81c, and the virtual ray 101d of the plurality of virtual rays 101 reaches another point of the second third object 81c.

Preferably, the number of virtual rays 101 which starts from a same point 46 is calibrated in the light field display parameters. The value of the numbers of virtual rays 100, 101 depends on the spatial and angular pitch, and the angle θₘₐₓ defining the maximum aperture of the light field 69.

For sake of clarity in figures 12 to 15, the virtual ray 100 is included in the plurality of rays noted 101.

In the example represented in figure 14, other pluralities of virtual rays are illustrated, and respectively noted 102, 103 and 104. These other pluralities of virtual rays 102, 103 and 104 start from different points of eye pupil plane 72 to points of the scene 80. These other pluralities of virtual rays 102, 103 and 104 reach points of the scene 80 that are similar or that differ from the points of the scene 80 reached by the plurality of virtual ray 101 illustrated in figure 12 and 11.

The examples represented in figure 12 to 14 allow scanning the model of the virtual lens 90 spatially according to the first and second directions 61,62 over the whole surface of the light field window 65 and angularly over the angular aperture of the light field window 65. In the model, each point and each angle of the light field display 60 is associated with one point of the scene 80 by a virtual ray. The corresponding points are calculated by taking into account the deviation angles when the virtual ray passes through the virtual lens 90, at the interface with each diopter 91, 92. It is noted that these examples have been divided for sake of clarity in order to explain the spatial and angular scan of the model of the virtual lens 90 that is carried out in the step of calculating 1004.

Thus, to reconstruct the virtual object 81 of the scene 80, the virtual objects 81 are divided into a plurality of virtual object points, each point of the scene 80 being optionally associated to a color and/or a luminosity.

In another embodiment, the plurality of virtual rays 101 in the step of calculating 1004 are configured to propagate from the objects 81 of the scene 80 to eye pupil plane 72. This is useful in particular if the propagation of the virtual rays is calculated on the base on wave optics.

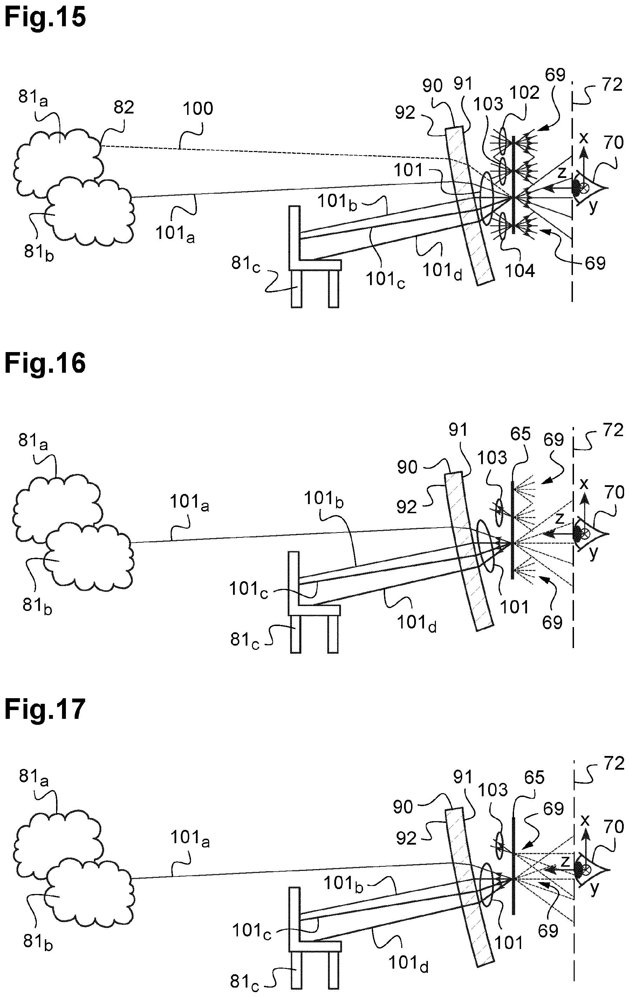

Figure 15 represents schematically an example of embodiment of the step of generating 1008.

After having calculated the virtual rays 100 and all the pluralities of virtual rays 101, 102, 103, 104, and determining, in the step of determining 1006, the real ray associated to these pluralities of virtual rays, the method 1000 or 2000 carries out the step of generating 1008. The step of generating 1008 is based on typical three-dimensions reconstruction method. In this example, a plurality of light fields 69 are generated towards the eye 70 of the subject 71. The light fields 69 generated are associated to all the virtual rays, i.e. the rays of plurality of virtual rays 101, 102, 103, 104, which have been calculated in the step of calculating 1004 and in the step of determining 1006. In this example, each light field 69 comprises a plurality of rays (real rays) having different directions of propagation. The virtual rays on the rear face of the light field 69 extend into real rays emerging from the front face of the light field window 65.

The real rays of the light field 69 are in the longitudinal extension of the virtual rays of the pluralities of virtual ray 101, 102, 103, 104 with respect to the light field window 65. The real rays allow the user to see the modified virtual three-dimensions scene 140, 83. The rays of the light field 69 comprise a color and/or a luminosity that correspond to the values determined in the step of calculating 1004 and in the step of generating 1006.

Figure 19 shows another example of the step of generating 1008. Reference 81a on figure 19 is associated to a virtual object 81 of the virtual scene 80, whereas the reference 83 corresponds to the image (i.e. displayed image) of the virtual object 81a through the light field display 60. The virtual lens 90 is illustrated for sake of clarity but in fact the optical effects of the virtual lens 90 are simulated by the light field display 60 (i.e. with the use of the light field display 60 according to the present disclosure, the wearer does not need the wear his ophthalmic glasses). In this example, the rays starting from a point of the virtual lens 90 are scattered towards the eye 70 of the subject according to a BTDF 150.

In the step of generating 1008, the light field display 60 generates for each virtual ray coming from the virtual object 81a a scattered beam according to the BTDF 150. Thus, each real ray of the light field 69 propagates towards the eye 70 according to a lobe of scattering corresponding to the BTDF 150. For example, the virtual ray 190a coming from a virtual object 81a in the virtual scene 80 is refracted according to the virtual lens 90 and scattered according to the BTDF 150a of the virtual lens 90 at the point numbered 193a where rays emitted from the object 81a pass through the virtual lens 90 (where the light is scattered). Another virtual ray 190b coming from the virtual object 81a is refracted according to the virtual lens 90 and scattered according the BTDF 150b at the point 193b positioned on the virtual lens 90.

In the example illustrated above, each BTDF 150 is represented as a circle comprises a main ray 692, which is for example numbered 692a for the main ray 692 associated to the virtual ray 190a of virtual object 81a, and which is numbered 692b for the main ray 692 associated to the virtual ray 190b of the virtual object 81a. The main ray 692 has the maximum probability. By main ray 692, it is meant the real ray 692 that is not scattered, the real ray that is only refracted. The BTDF 150 also comprises secondary rays 693, which are numbered 693a for secondary rays associated to the virtual ray 190a, and which are numbered 693b for the virtual ray 190b. The secondary rays 693 have reduced intensity or reduced probability compared to the main ray 692. Each ray of the BTDF 150 reaches the eye pupil plane 72. The BTDF 150 varies spatially and angularly along the surface of the virtual lens 90. Thus, it means the secondary ray 693b associated to the virtual ray 190b will not have necessarily the same probability or intensity of the secondary ray 693a associated to the virtual ray 190a. For example, at the point 193b of the first diopter 91, several emerging secondary rays 693b are generated by the light field display 60 in various directions, with a probability depending on the BTDF 150b. The rays 194 linked to the image 83 are a schematic representation of the rays emit by the light field display 60 to simulate the effects of the corrective lens of the subject 71.

According to the method of the disclosure, the BTDF is preferably considered in the step of calculating 1004 and determining 1006 in order to be generated by the light field display 60 in the step of generating 1008 according to the BTDF 150.

Figure 20 shows another example of the step of generating 1008. In this example, the virtual lens 90 is positioned between the light field window 65 and the eye 70 of the subject 71.

To determine the value of a real ray 700 generated from a point of the light field window 65 and with a given angle between the real ray 700 and the plane of the light field display 60 to a point of the eye pupil plane 72, the step of calculating 1004 is configured to determine a virtual ray 701, associated to the real ray 700, starting from the point of the eye pupil plane 72. Then, the virtual ray 701 is extended toward the virtual scene 80. To consider the model of the virtual lens 90, the step of calculating 1004 calculates the virtual ray 701 that is deviated by the virtual lens 90 and then extends it to the virtual scene 80. The virtual ray 701 reaches the virtual object 81a at the point 82 that has (x, y, z) coordinates. Each point of the object 81a of the virtual scene 80 is associated to a color and/or a luminosity. Thus, the point 82 is associated to a color and/or a luminosity. Consequently, in the step of generating 1008, the value of the real ray 700 generated by the light field display 60, which is associated to the virtual ray 701, will be then the value of the color and/or luminosity of the point 82. Then, this operation is repeated with other real rays, for example with the real ray numbered 703 associated to the virtual ray 704 and the point object numbered 84, in order to scan spatially and angularly the light field display 60.

Figures 16, respectively 17 represent schematically examples of embodiment of the step of selecting applied in the step of calculating 1004 and/or respectively in the step of generating 1008.

Figure 16 shows a schematic representation of the step of selecting 2006 applied in the step of calculating 1004.

In this example, the step of calculating 1004 only calculates virtual rays that reach the eye 70 of the subject 71. For example, the plurality of virtual rays noted 103 of figure 16 comprises less virtual rays than the plurality of virtual rays noted 103 shown in figure 14. In addition, the virtual ray noted 100 has been deleted of figure 16 because the ray (real ray) associated to this virtual ray 100 doesn't reach the eye 70 of the subject 71. Thus, the step of calculating 1004 is less time consuming.

This step is, for example, implemented by calculating a ray (virtual ray) that reaches the eye 70 of the subject 71 at a first end and, at a second end, that reaches a point of the light field window 65 oriented in the first side 66 of the light field display 60. This ray may also be associated to a virtual ray located on the second side 67 of the light field display 60. If this ray does not reach the eye 70 of the subject 71, then the virtual ray associated (stating from the second side 67 of the light field display) is not considered in the step of calculation 1004.

Following the step of selecting 2006 applied to the step of calculating 1004, the method according to the current disclosure advantageously performs the step of selecting 2008 applied to the step of generating 1008.

In that case, only the real rays of the light field 69 that reach the eye 70 of the subject 71 are calculated in the step of determining 1006. This selection allows only generating light field 69 comprising real rays that reach the eye 70 of the subject 71 in the step of generating 1008. For example, in figure 17, the generated light field 69 comprises only real rays that reach the eye 70 of the subject 71. These real rays correspond to the virtual rays that have been selected in the step of selecting 2006 applying in the step of calculating 1004. Consequently, the method according to the current disclosure is optimized because the steps of calculating 1004, determining 1006 and generating 1008 are less time consuming and consumes less energy. In addition, it provides a better visual comfort.

### Device

Figure 18 shows a subject 71 viewing a modified virtual three-dimensions scene 140 according to the method 1200 or 2000. The modified virtual three-dimension scene 140 corresponds to the virtual three-dimensions scene 80 that is really displayed to the subject 71. The modified virtual three-dimensions scene 140 comprises all the elements included in the virtual three-dimensions scene 80.

In this example, the subject 71 views the modified virtual three-dimensions scene 140 through a system 130. The system 130 is a virtual reality (VR) device or an augmented reality (AR) device 130 that is worn by the subject 71. In this example, the light field display 60 is included into the system 130. Specifically, the light field display 60 is arranged to be positioned in front of the eyes of the subject 71.

The modified virtual three-dimensions scene 140 illustrated in figure 18 is a representation of an image of a scene of the current life of the subject 71. The modified virtual three-dimensions scene 140 is seen clearly by the subject 71. The vision of the subject 71 is thus corrected.

The system 130 is connected to a computer 120 or calculation module 120 or a calculator 120 arranged to control the light field display 60. In this example, the calculator 120 is embedded into the system 130. In another embodiment, the calculator 120 is remotely connected to the system 130.

The calculator 120 is configured to perform at least the step of receiving 1002, calculating 1004, determining 1006 of the method 1200 or 2000 according to the current disclosure. The light field display 60 and the calculator 120 are arranged to carry out the step of generating 1008.

The system 130 is adapted and configured to operate the method 1000 and 2000.

In an embodiment, the light field display 60 is used for the two eyes 70 of the subject 71.

In another embodiment, the system 130 comprises two light field displays 60 associated each respectively with each of the two eyes 70 of the subject 71. The system 130 is configured to generate and to display two virtual modified three-dimensions scenes 140 allowing to obtain a binocular vision.

In another embodiment, the system 130 may be an augmented reality device 130. Thus, the light field display 60 is arranged on real lenses in order to directly project the modified virtual three-dimensions 140 on the real lenses of the subject. The system 130 is, for example, augmented reality glasses.

## Claims

1. Method for simulating an ophthalmic lens on an eye of a subject viewing a virtual three-dimensions scene using a light field display, said light field display comprising a light field window, said method comprising the following steps:
- receiving a set of input parameters comprising scene parameters of the virtual three-dimensions scene,
- calculating, by ray tracing, a virtual ray between a point of the virtual three-dimensions scene and a point of an eye pupil plane, the virtual ray passing through a virtual lens and being defined on the basis of a model providing a deviation angle of the virtual ray through the virtual lens, the deviation angle depending on at least one incidence angle and position of the virtual ray on the virtual lens, repeating the calculating by ray tracing for a plurality of virtual rays passing through the virtual lens and joining couples of one point of the virtual three-dimensions scene and another point of the eye pupil plane, so as to scan spatially and angularly the light field window;
- determining a light field representing a modified virtual three-dimensions scene based on the scene parameters and on the deviation angle associated to each virtual ray of the plurality of virtual rays;
- generating the light field representing the modified virtual three-dimensions scene on the light field window towards the eye pupil plane of the subject.

2. Method according to claim 1 wherein the light field window corresponds to a field of view of at least 40 degrees in a horizontal plane and 40 degrees in a vertical plane and/or an angular resolution of less than 5 degrees.

3. Method according to claim 1 or 2 wherein the light field window extends spatially over a surface area of at least 3 mm in a horizontal plane and 3 mm in a vertical plane and/or a spatial resolution of at least 0,5 dot/mm.

4. Method according to any one of claims 1 to 3 comprising a step of acquiring a three-dimensions image of the scene using a three-dimensions capturing device to extract the scene parameters, said scene parameters comprising geometric and/or photometric properties of the scene.

5. Method according to claim 4 wherein the step of determining a light field comprises a step of applying the geometric and/or photometric properties of the scene.

6. Method according to any one of claim 1 to 5 wherein the step of calculating and/or the step of generating comprises a step of selecting a subset of the plurality of virtual rays passing through the pupil of the eye of the subject.

7. Method according to any one of claim 1 to 6 wherein the model is determined on the basis of geometrical optics calculations and/or wave optics calculations and/or photometric optics calculation.

8. Method according to any one of claim 1 to 7 wherein the model comprises a shape of the lens and a positioning of the lens in relation to the eye.

9. Method according to any one of claim 1 to 8 wherein the model comprises distortion model of the virtual lens and/or a spherical power model of the virtual lens comprising at least a sphere parameter, and/or an astigmatism power model of the virtual lens comprising at least a cylinder and/or an axis parameters or J0 and J45 parameters and/or a filtering lens.

10. Method according to any one of claim 1 to 9 wherein the step of determining comprises a step of representing the modified virtual three-dimensions scene further comprising applying a convolution or image deformation, adapted for simulating blur, distortion, diffraction, chromatism, scattering or transmission attenuation.

11. Method according to any one of claim 1 to 10 wherein the method is adapted for simulating an other ophthalmic lens on an other eye of the subject, said method comprising the steps of calculating, determining and generating for simulating the other ophthalmic lens for the other eye of the subject, said subject parameters in the step of receiving comprising other parameters among a pupil diameter of the other eye and/or a pupil position of the other eye relative to the light field display.

12. Method according to any one of claim 1 to 11 wherein the virtual lens comprises a progressive power lens or a multifocal lens, a single vision lens, an eyeglass lens, a contact lens or a filter.

13. Method according to any one of claim 1 to 12 wherein the virtual ray and the plurality of virtual rays in the step of calculating are configured to propagate from the eye pupil plane to the at least one object of the scene.

14. Method according to any one of claim 1 to 12 wherein the virtual ray and the plurality of virtual rays in the step of calculating are configured to propagate from the at least one object of the scene to the eye pupil plane.

15. System comprising a calculator and a light field display, the system being adapted and configured to operate the method according to any one of claims 1 to 14.

## Patentansprüche

1. Verfahren zum Simulieren einer ophthalmischen Linse auf einem Auge einer Zielperson, die eine virtuelle dreidimensionale Szene unter Verwendung einer Lichtfeldanzeige betrachtet, wobei die Lichtfeldanzeige ein Lichtfeldfenster umfasst, wobei das Verfahren die folgenden Schritte umfasst:
- Empfangen eines Satzes von Eingabeparametern, die Szenenparameter der virtuellen dreidimensionalen Szene umfassen,
- Berechnen, durch Strahlverfolgung, eines virtuellen Strahls zwischen einem Punkt der virtuellen dreidimensionalen Szene und einem Punkt einer Augenpupillenebene, wobei der virtuelle Strahl durch eine virtuelle Linse verläuft und basierend auf einem Modell definiert wird, das einen Abweichungswinkel des virtuellen Strahls durch die virtuelle Linse bereitstellt, wobei der Abweichungswinkel von wenigstens einem Einfallswinkel und einer Position des virtuellen Strahls auf der virtuellen Linse abhängt, wobei das Berechnen durch Strahlverfolgung für mehrere virtuelle Strahlen wiederholt wird, die durch die virtuelle Linse verlaufen und Paare aus einem Punkt der virtuellen dreidimensionalen Szene und einem anderen Punkt der Augenpupillenebene verbinden, sodass das Lichtfeldfenster räumlich und winkelbasiert gescannt wird;
- Bestimmen eines Lichtfelds, das eine modifizierte virtuelle dreidimensionale Szene repräsentiert, basierend auf den Szenenparametern und auf dem Abweichungswinkel, der mit jedem virtuellen Strahl der mehreren virtuellen Strahlen assoziiert ist;
- Erzeugen des Lichtfeldes, das die modifizierte virtuelle dreidimensionale Szene repräsentiert, auf dem Lichtfeldfenster zu der Augenpupillenebene der Zielperson hin.

2. Verfahren nach Anspruch 1, wobei das Lichtfeldfenster einem Sichtfeld von wenigstens 40 Grad in einer horizontalen Ebene und 40 Grad in einer vertikalen Ebene und/oder einer Winkelauflösung von weniger als 5 Grad entspricht.

3. Verfahren nach Anspruch 1 oder 2, wobei sich das Lichtfeldfenster räumlich über einen Oberflächenbereich von wenigstens 3 mm in einer horizontalen Ebene und 3 mm in einer vertikalen Ebene und/oder eine räumliche Auflösung von wenigstens 0,5 Punkte/mm erstreckt.

4. Verfahren nach einem der Ansprüche 1 bis 3, das einen Schritt des Erfassens eines dreidimensionalen Bildes der Szene unter Verwendung einer dreidimensionalen Erfassungsvorrichtung zum Extrahieren der Szenenparameter umfasst, wobei die Szenenparameter geometrische und/oder photometrische Eigenschaften der Szene umfassen.

5. Verfahren nach Anspruch 4, wobei der Schritt des Bestimmens eines Lichtfeldes einen Schritt des Anwendens der geometrischen und/oder photometrischen Eigenschaften der Szene umfasst.

6. Verfahren nach einem der Ansprüche 1 bis 5, wobei der Schritt des Berechnens und/oder der Schritt des Erzeugens einen Schritt des Auswählens einer Teilmenge der mehreren virtuellen Strahlen umfasst, die durch die Pupille des Auges der Zielperson verlaufen.

7. Verfahren nach einem der Ansprüche 1 bis 6, wobei das Modell basierend auf Berechnungen der geometrischen Optik und/oder Berechnungen der Wellenoptik und/oder einer Berechnung der photometrischen Optik bestimmt wird.

8. Verfahren nach einem der Ansprüche 1 bis 7, wobei das Modell eine Form der Linse und eine Positionierung der Linse in Bezug auf das Auge umfasst.

9. Verfahren nach einem der Ansprüche 1 bis 8, wobei das Modell ein Verzerrungsmodell der virtuellen Linse und/oder ein Modell einer sphärischen Brechkraft der virtuellen Linse, das wenigstens einen Sphärenparameter umfasst, und/oder ein Modell einer Astigmatismusbrechkraft der virtuellen Linse, das wenigstens einen Zylinder- und/oder Achsparameter oder J0- und J45-Parameter umfasst, und/oder eine Filterlinse umfasst.

10. Verfahren nach einem der Ansprüche 1 bis 9, wobei der Schritt des Bestimmens einen Schritt des Repräsentierens der modifizierten virtuellen dreidimensionalen Szene umfasst, der ferner Anwenden einer Faltung oder Bilddeformation umfasst, die zum Simulieren von Unschärfe, Verzerrung, Beugung, Chromatismus, Streuung oder Transmissionsabschwächung eingerichtet ist.

11. Verfahren nach einem der Ansprüche 1 bis 10, wobei das Verfahren zum Simulieren einer anderen ophthalmischen Linse auf einem anderen Auge der Zielperson eingerichtet ist, wobei das Verfahren die Schritte des Berechnens, Bestimmens und Erzeugens zum Simulieren der anderen ophthalmischen Linse für das andere Auge der Zielperson umfasst, wobei die Zielpersonenparameter in dem Schritt des Empfangens andere Parameter unter einem Pupillendurchmesser des anderen Auges und/oder einer Pupillenposition des anderen Auges relativ zu der Lichtfeldanzeige umfassen.

12. Verfahren nach einem der Ansprüche 1 bis 11, wobei die virtuelle Linse eine Gleitsichtlinse oder eine Multifokallinse, eine Einstärkenlinse, eine Brillenglaslinse, eine Kontaktlinse oder ein Filter umfasst.

13. Verfahren nach einem der Ansprüche 1 bis 12, wobei der virtuelle Strahl und die mehreren virtuellen Strahlen in dem Schritt des Berechnens dazu konfiguriert sind, von der Augenpupillenebene zu dem wenigstens einen Objekt der Szene zu propagieren.

14. Verfahren nach einem der Ansprüche 1 bis 12, wobei der virtuelle Strahl und die mehreren virtuellen Strahlen in dem Schritt des Berechnens dazu konfiguriert sind, von dem wenigstens einen Objekt der Szene zu der Augenpupillenebene zu propagieren.

15. System, das eine Rechenmaschine und eine Lichtfeldanzeige umfasst, wobei das System dazu eingerichtet und konfiguriert ist, das Verfahren nach einem der Ansprüche 1 bis 14 durchzuführen.

## Revendications

1. Procédé de simulation d'une lentille ophtalmique sur un œil d'un sujet regardant une scène virtuelle tridimensionnelle au moyen d'un dispositif d'affichage de champ lumineux, ledit dispositif d'affichage de champ lumineux comprenant une fenêtre de champ lumineux, ledit procédé comprenant les étapes suivantes :
- réception d'un ensemble de paramètres d'entrée comprenant des paramètres de scène de la scène virtuelle tridimensionnelle ;
- calcul, par lancer de rayons, d'un rayon virtuel entre un point de la scène virtuelle tridimensionnelle et un point d'un plan pupillaire de l'œil, le rayon virtuel traversant une lentille virtuelle et étant défini sur la base d'un modèle fournissant un angle de déviation du rayon virtuel à travers la lentille virtuelle, l'angle de déviation dépendant d'au moins un angle d'incidence et d'une position du rayon virtuel sur la lentille virtuelle, ce calcul par lancer de rayons étant répété pour une pluralité de rayons virtuels traversant la lentille virtuelle et reliant des couples constitués d'un point de la scène virtuelle tridimensionnelle et d'un autre point du plan pupillaire de l'œil, de manière à balayer spatialement et angulairement la fenêtre de champ lumineux ;
- détermination d'un champ lumineux représentant une scène virtuelle tridimensionnelle modifiée sur la base des paramètres de scène et de l'angle de déviation associé à chaque rayon virtuel de la pluralité de rayons virtuels ;
- génération, sur la fenêtre de champ lumineux en direction du plan pupillaire de l'œil du sujet, du champ lumineux représentant la scène virtuelle tridimensionnelle modifiée.

2. Procédé selon la revendication 1, dans lequel la fenêtre de champ lumineux correspond à un champ de vision d'au moins 40 degrés dans un plan horizontal et 40 degrés dans un plan vertical et/ou à une résolution angulaire inférieure à 5 degrés.

3. Procédé selon la revendication 1 ou 2, dans lequel la fenêtre de champ lumineux s'étend spatialement sur une surface d'au moins 3 mm dans un plan horizontal et 3 mm dans un plan vertical et/ou une résolution spatiale d'au moins 0,5 point/mm.

4. Procédé selon l'une quelconque des revendications 1 à 3, comprenant une étape d'acquisition d'une image tridimensionnelle de la scène au moyen d'un dispositif de capture tridimensionnelle afin d'extraire les paramètres de scène, lesdits paramètres de scène comprenant des propriétés géométriques et/ou photométriques de la scène.

5. Procédé selon la revendication 4, dans lequel l'étape de détermination d'un champ lumineux comprend une étape d'application des propriétés géométriques et/ou photométriques de la scène.

6. Procédé selon l'une quelconque des revendications 1 à 5, dans lequel l'étape de calcul et/ou l'étape de génération comprennent une étape de sélection d'un sous-ensemble de la pluralité de rayons virtuels traversant la pupille de l'œil du sujet.

7. Procédé selon l'une quelconque des revendications 1 à 6, dans lequel le modèle est déterminé sur la base de calculs d'optique géométrique et/ou de calculs d'optique ondulatoire et/ou de calculs d'optique photométrique.

8. Procédé selon l'une quelconque des revendications 1 à 7, dans lequel le modèle comprend une forme de la lentille et un positionnement de la lentille par rapport à l'œil.

9. Procédé selon l'une quelconque des revendications 1 à 8, dans lequel le modèle comprend un modèle de distorsion de la lentille virtuelle et/ou un modèle de puissance sphérique de la lentille virtuelle comprenant au moins un paramètre de sphère, et/ou un modèle de puissance d'astigmatisme de la lentille virtuelle comprenant au moins des paramètres de cylindre et/ou d'axe ou des paramètres J0 et J45 et/ou une lentille filtrante.

10. Procédé selon l'une quelconque des revendications 1 à 9, dans lequel l'étape de détermination comprend une étape de représentation de la scène virtuelle tridimensionnelle modifiée comprenant en outre l'application d'une convolution ou d'une déformation d'image adaptée à la simulation d'un flou, d'une distorsion, d'une diffraction, d'un chromatisme, d'une diffusion ou d'une atténuation de transmission.

11. Procédé selon l'une quelconque des revendications 1 à 10, le procédé étant adapté à la simulation d'une autre lentille ophtalmique sur un autre œil du sujet, ledit procédé comprenant les étapes de calcul, de détermination et de génération pour la simulation de l'autre lentille ophtalmique pour l'autre œil du sujet, lesdits paramètres du sujet à l'étape de réception comprenant d'autres paramètres parmi un diamètre pupillaire de l'autre œil et/ou une position pupillaire de l'autre œil par rapport au dispositif d'affichage de champ lumineux.

12. Procédé selon l'une quelconque des revendications 1 à 11, dans lequel la lentille virtuelle comprend une lentille à puissance progressive ou une lentille multifocale, une lentille monofocale, un verre de lunettes, une lentille de contact ou un filtre.

13. Procédé selon l'une quelconque des revendications 1 à 12, dans lequel le rayon virtuel et la pluralité de rayons virtuels à l'étape de calcul sont configurés pour se propager depuis le plan pupillaire de l'œil jusqu'à l'au moins un objet de la scène.

14. Procédé selon l'une quelconque des revendications 1 à 12, dans lequel le rayon virtuel et la pluralité de rayons virtuels à l'étape de calcul sont configurés pour se propager depuis l'au moins un objet de la scène jusqu'au plan pupillaire de l'œil.

15. Système comprenant un calculateur et un dispositif d'affichage de champ lumineux, le système étant adapté et configuré pour mettre en œuvre le procédé selon l'une quelconque des revendications 1 à 14.
